# EUROPEAN PATENT APPLICATION

(11) **EP 4 491 618 A1**
(43) Date of publication of application: **15.01.2025**
(21) Application number: 23767081.5
(22) Date of filing: 02.03.2023
(51) Int. Cl.: C07D 263/57, A61K 31/423

(54) **NOVEL TAFAMIDIS SYNTHESIS METHOD**

(30) Priority: 10.03.2022 KR 20220030262
(71) Applicant: Korea University Research and Business Foundation, Seoul 02841 (KR)
(72) Inventor: CHEON, Cheol-Hong, Seoul 04983 (KR); PARK, Jinjae, Seoul 02843 (KR); KIM, Jong Mu, Seoul 02842 (KR)
(74) Representative: Pfenning, Meinig & Partner mbB
(86) International application number: PCT/KR2023/002888
(87) International publication number: WO 2023/171980

(57) **Abstract**

The present invention relates to a novel manufacturing method for tafamidis with excellent synthesis yield and cost-effectiveness. More specifically, the invention involves synthesizing tafamidis with a benzoxazole backbone by reacting 4-amino-3-hydroxybenzoic acid and 3,5-dichlorobenzaldehyde to form an imine, followed by oxidation cyclization using a cyanide anion-containing catalyst under basic conditions in air. This method excludes the use of additional oxidizing agents such as conventional metal catalysts and/or high concentrations of oxygen, while minimizing separation processes, resulting in a novel synthesis method for tafamidis with excellent synthesis yields.

## Description

### [Field of the Invention]

The present invention relates to a novel manufacturing method for tafamidis with excellent synthesis yield and cost-effectiveness. More specifically, the invention involves synthesizing tafamidis with a benzoxazole backbone by reacting 4-amino-3-hydroxybenzoic acid and 3,5-dichlorobenzaldehyde to form an imine, followed by oxidation cyclization using a cyanide anion-containing catalyst under basic conditions in air. This method excludes the use of additional oxidizing agents such as conventional metal catalysts and/or high concentrations of oxygen, while minimizing separation processes, resulting in a novel synthesis method for tafamidis with excellent synthesis yields.

### [Background of the Invention]

Tafamidis (brand names: Vyndaqel or Vyndamax) is a drug used to delay the progression of transthyretin amyloidosis in adults with certain forms of the disease. It is used to treat hereditary forms such as familial amyloid cardiomyopathy and familial amyloid polyneuropathy, as well as the previously known senile systemic amyloidosis, which is wild-type transthyretin amyloidosis. In individuals with transthyretin amyloidosis, transthyretin disassembles and forms amyloid deposits that damage tissues, including nerves and the heart. Tafamidis works by stabilizing the quaternary structure of the transthyretin protein, thereby delaying the progression of these diseases.

Tafamidis was first developed by Professor Jeffery W. Kelly and his team at the Scripps Research Institute in 2003. The same year, Professor Kelly's team, together with Professor Susan Lindquist from MIT, founded FoldRx, which was acquired by Pfizer in 2010 for further development. After receiving approval from the EMA (European Medicines Agency) in 2011, tafamidis was also approved for use in Japan in 2013. In 2019, it received FDA approval for transthyretin-related amyloid cardiomyopathy. By 2020, it had generated worldwide sales of approximately 1.29 billion dollars, demonstrating significant market potential. Given the anticipated increase in prevalence and prescriptions, the related market is expected to grow further. While the substance patent for tafamidis varies by country, it is generally set to expire between late 2023 and early 2024. Consequently, there is significant interest and active research in generic synthesis of this compound by various pharmaceutical companies, including those in South Korea.

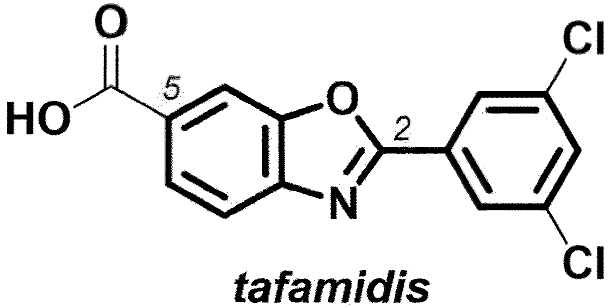

Tafamidis structurally features a benzoxazole backbone with a 3,5-dichlorophenyl group at position 2 of benzoxazole and a highly polar carboxyl group at position 5 of benzoxazole. It is currently commercially produced through the pathway developed by Professor Kelly's team in 2003, as illustrated in Scheme 1 below.

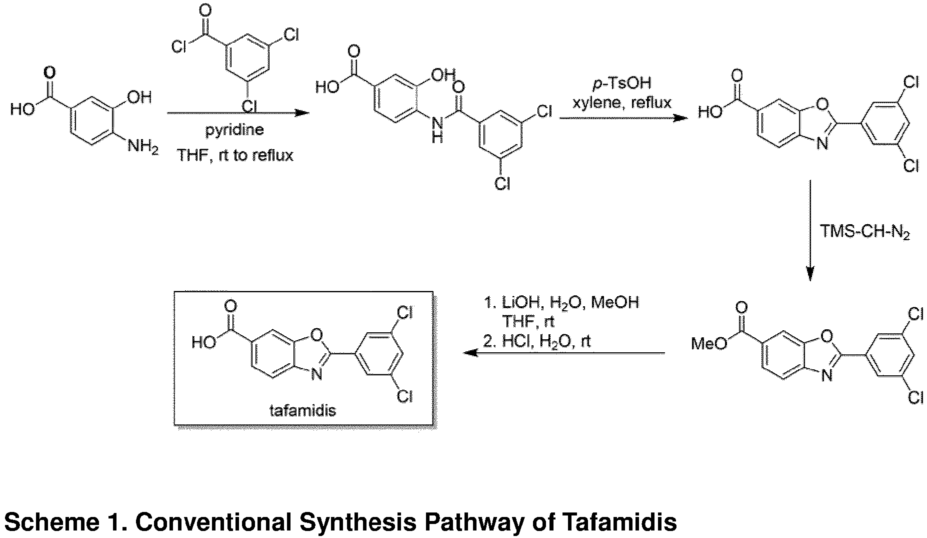

Initially, 4-amino-3-hydroxybenzoic acid is reacted with 3,5-dichlorobenzoyl chloride to form an amide. This amide undergoes a dehydration cyclization reaction under acidic conditions to produce Tafamidis with a benzoxazole backbone. Subsequently, the carboxyl group is converted into a methyl ester group by reacting with diazomethane, and the ester group is hydrolyzed to yield the final compound, Tafamidis. However, this synthesis method mentioned above involves the unnecessary step of converting the carboxyl group to an ester group and then back to a carboxyl group through hydrolysis. Additionally, the esterification reaction requires the use of diazomethane, which presents issues, and moreover, the overall yield is as low as around 10%, indicating that significant improvements are needed.

On the other hand, Ferlin et al. have reported a method for synthesizing benzoxazoles after forming an imine in their literature (Francesco Ferlin, et al., Continuous flow/waste-minimized synthesis of benzoxazoles catalyzed by heterogeneous manganese systems, Green Chem., 2019, 21, 5298). However, their method improves reactivity by using different types of catalysts, specifically heterogeneous mixed valence manganese octahedral molecular sieves (OMSs), along with high-concentration oxygen (100% oxygen), in the method for producing benzoxazoles through oxidation reactions which involves using conventional metal catalysts.

This method differs from the present invention, which does not use metal catalysts or high-concentration oxygen.

The inventors of the present invention solved problems of the conventional manufacturing method for tafamidis by reacting 4-amino-3-hydroxybenzoic acid with 3,5-dichlorobenzaldehyde to form an imine, and then using a cyanide anion-containing catalyst under basic conditions in air to carry out an oxidative cyclization reaction to synthesize tafamidis with a benzoxazole backbone. They discovered that this approach offers significant advantages in terms of both cost-effectiveness and yield, leading to the completion of the present invention.

### [Detailed Description of the Invention]

### [Technical Problem]

The objective of the present invention is to provide a novel method for manufacturing tafamidis that excludes metal catalysts used in the conventional manufacturing method for tafamidis and does not require additional oxidizing agents such as high-concentration oxygen. This eliminates the need to remove residual metals and allows the reaction to be performed in air.

Another objective of the present invention is to provide a novel method for manufacturing tafamidis that allows for cost-effectiveness with excellent yield and purity through a single separation process.

### [Technical Solution]

To achieve the above objectives, one embodiment of the invention provides a manufacturing method of compound 1 characterized by comprising:
(a) Obtaining compound S1 by reacting compound 2 with compound 3;
(b) Converting compound S1 to compound S1' using 1 equivalent of a base containing M;
(c) Converting compound S1' to compound S2 using M'CN as a catalyst under basic conditions; and
(d) Converting compound S2 to compound 1 by acid treatment.

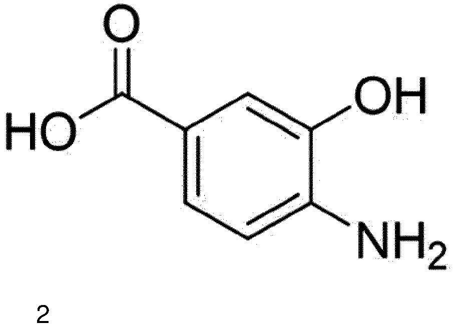

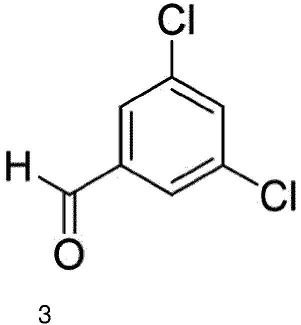

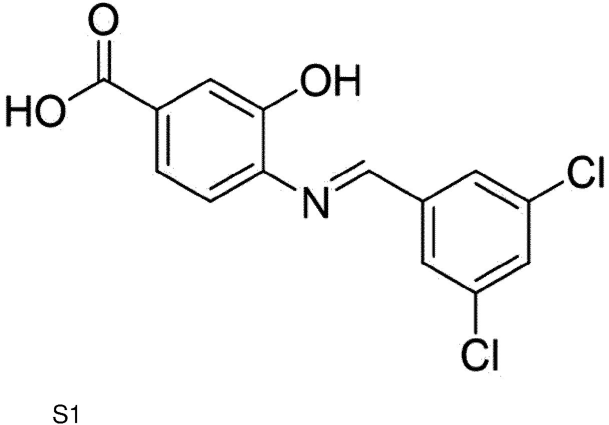

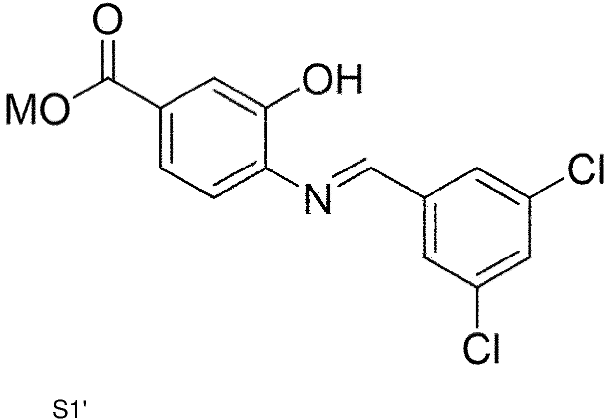

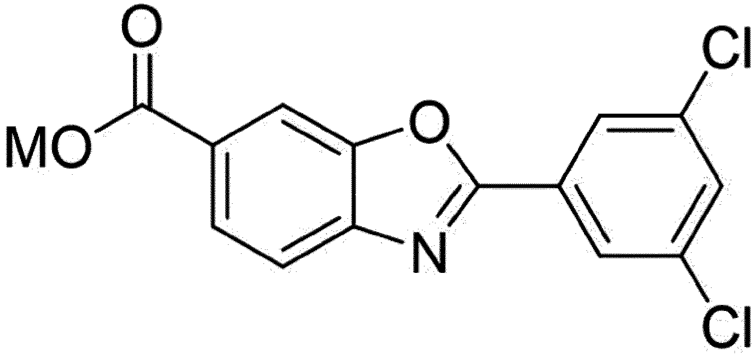

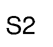

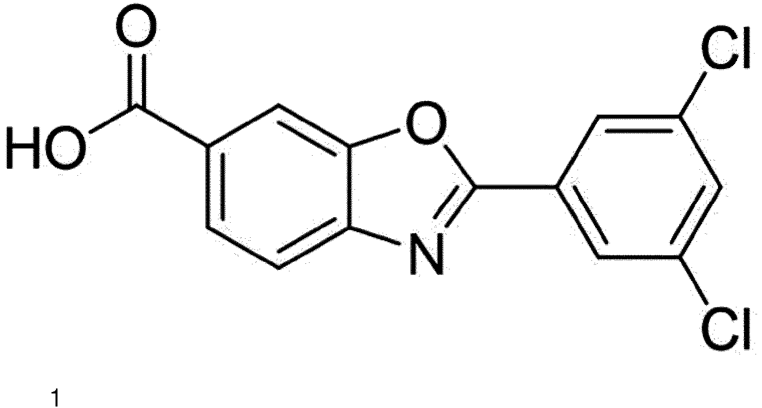

Where M and M' are each independently selected from the group consisting of alkali metals, alkaline earth metals, and ammonium, and the manufacturing method is performed in air without using metal catalysts and/or additional oxidizing agents.

In one embodiment of the present invention, the step (a) of obtaining compound S1 is preferably performed in the presence of a dehydrating agent. The use of such dehydrating agents removes water molecule generated during imine intermediate formation, thereby facilitating the overall reaction. Examples of suitable dehydrating agents which can be used in the present invention include one or more compounds selected from the group consisting of MgSO₄ and Na₂SO₄ but are not limited herein. When TiCl₄ is used as a dehydrating agent in one embodiment of the present invention, a base must be used, wherein a preferred example of the base is Et₃N.

In one embodiment of the present invention, step (a) can be carried out using molecular sieves or azeotropic distillation.

Alternatively, in one embodiment of the present invention, step (a) can be performed using a Dean-Stark apparatus.

The term "alkali metals" used herein refers to Group 1 elements of the periodic table: the chemical elements consisting of lithium (Li), sodium (Na), potassium (K), rubidium (Rb), cesium (Cs), and others.

The term "alkaline earth metals" used herein refers to Group 2 elements of the periodic table: the chemical elements consisting of beryllium (Be), magnesium (Mg), calcium (Ca), strontium (Sr), barium (Ba) , and others.

The term "base containing M" used herein refers to an inorganic base containing M, selected from the group consisting of alkali metals, alkaline earth metals, and ammonium, which can convert compound S1 to compound S1' through an acid-base reaction. Specifically, an inorganic base is a non-organic base, which refers to salts containing M's protons such as hydroxide salts, carbonate salts, phosphate salts, cyanide salts, but are not limited herein.

In one embodiment of the present invention, M is preferably an alkali metal.

Preferred examples of "base containing M" used in one embodiment of the present invention include lithium hydroxide, sodium hydroxide, potassium hydroxide, rubidium hydroxide, cesium hydroxide, ammonium hydroxide, lithium carbonate, sodium carbonate, potassium carbonate, rubidium carbonate, cesium carbonate, ammonium carbonate, lithium phosphate, sodium phosphate, potassium phosphate, rubidium phosphate, cesium phosphate, ammonium phosphate, lithium cyanide, sodium cyanide, potassium cyanide, rubidium cyanide, cesium cyanide, ammonium cyanide, lithium acetate, sodium acetate, potassium acetate, rubidium acetate, cesium acetate, ammonium acetate, but are not limited herein.

In one embodiment of the present invention, the base is used in the presence of a phase-transfer catalyst. Alternatively, in another embodiment, the base can be used in the presence of an organic solvent. In another embodiment, the base can be used in the presence of one or more of a phase-transfer catalyst, water, or an organic solvent.

The term "cyanide anion-containing catalyst" used herein is represented by M'CN, where M' is selected from the group consisting of alkali metals, alkaline earth metals, and ammonium.

In one embodiment of the present invention, step (c) involves forming a benzoxazole ring through oxidation cyclization using a cyanide anion-containing catalyst under basic conditions in air, thus eliminating the need for metal catalysts and additional oxidizing agents such as high concentrations of oxygen. Preferred examples of cyanide anion-containing catalysts include sodium cyanide or potassium cyanide but are not limited herein.

In one embodiment of the present invention, some cyanide anion-containing catalysts can serve as both base and catalyst. For example, when sodium cyanide is used as both base and catalyst, 1.1 equivalents of sodium cyanide are used, allowing both steps (b) and (c) to be carried out.

In one embodiment of the present invention, any suitable solvent can be used in steps (b) and (c). Specifically, polar aprotic solvents are preferred for steps (b) and (c). Examples of suitable polar aprotic solvents used in one embodiment of the present invention include acetone, acetonitrile, tetrahydrofuran, dimethyl sulfoxide (DMSO), dimethylformamide (DMF), and hexamethylphosphoramide (HMPA), but are not limited herein. In one embodiment of the present invention, DMF or DMSO is preferred as the polar aprotic solvent used in steps (b) and (c).

In one embodiment of the present invention, step (c) is preferably carried out under basic conditions.

In one embodiment of the present invention, step (c) can be performed at any suitable temperature. For example, a suitable temperature for step (c) is above room temperature, and may range from 25°C to 100°C, or from 25°C to 80°C, or from 50°C to 80°C.

In one embodiment of the present invention, the compound S1 obtained in step (a) can be separated through a separation process as judged by a person skilled in the art, or it can be used directly in step (b) without a separate separation process. For example, in the present invention, it is preferable to use the compound S1 obtained in step (a) directly in step (b) without a separation process, which allows for cost-effective synthesis of tafamidis with excellent yield and purity.

In one embodiment of the present invention, step (d) involves the final treatment of the compound S2 obtained under basic conditions with an acid to obtain tafamidis. There is no limitation on the type of acid used in the present invention, but preferably sulfuric acid or hydrochloric acid is used.

The term "air" used herein refers to the colorless and transparent gas that constitutes the lower part of the atmosphere surrounding the Earth, which includes nitrogen and oxygen as the main components, as well as small amounts of carbon dioxide, argon, and other gases.

The reaction mixtures at each step of the present invention can be at any suitable pressure. Such pressure typically affects the reaction rate, the state of the reactants, and other factors. For example, it is preferable that the reaction mixture of the present invention be conducted at normal pressure (1 atm) without applying additional pressure, but this is not limited. For examples, the reaction mixtures at each step of the present invention can be conducted at relatively low or high pressure conditions, as long as it does not affect the overall reaction yield. However, while compressed air may be used in the present invention, high-concentration oxygen (100% oxygen) is not used.

### [Effects of the Invention]

According to the present invention, a novel method for synthesizing tafamidis is provided, which excludes metal catalysts used in the conventional manufacturing method for tafamidis and does not require additional oxidizing agents such as high-concentration oxygen. This eliminates the need for a process to remove residual metals and allows the reaction to be performed in air.

Furthermore, the present invention provides a novel method for manufacturing tafamidis that allows for cost-effectiveness with excellent yield and purity through a single separation process.

### [Brief Description of the Drawings]

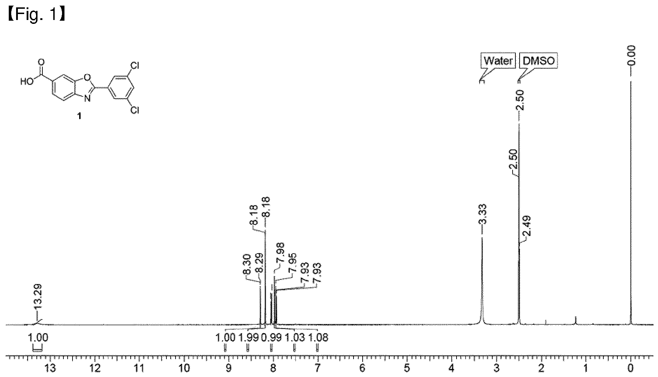

Figure 1 shows the NMR spectrum of tafamidis 1.

### [Description of the Embodiments of the Invention]

The following examples will provide a more detailed description of the present invention. However, these examples are provided for the purpose of illustrating the invention and do not limit the scope of the invention.

### General Procedure

Unless otherwise stated, all reactions were performed in oven-dried glassware under an argon atmosphere. Unless otherwise indicated, all reactions were stirred magnetically and monitored by thin-layer chromatography (TLC) using pre-coated silica gel glass plates (0.25 mm) with F254 indicator and visualized under UV light (254 nm). Flash column chromatography was performed using silica gel 60 (230 - 400 mesh) with the indicated eluent. Commercial grade reagents were used without further purification. Unless otherwise specified, yields refer to chromatographically and spectroscopically pure compounds. ¹H NMR and ¹³C NMR spectra were recorded on 500 MHz and 125 MHz spectrometers, respectively. Tetramethylsilane (δTMS: 0.0 ppm) and residual NMR solvents (CDCl₃ (δH: 7.26 ppm, δC: 77.16 ppm) or (CD₃ )₂SO (δH: 2.50 ppm, δC: 39.52 ppm)) were used as internal standards for ¹H NMR and ¹³C NMR spectra, respectively. Proton spectra are indicated as δ (proton position, multiplicity, coupling constant J, number of protons). Multiplicity is indicated as s (singlet), d (doublet), t (triplet), q (quartet), p (quintet), m (multiplet), and br (broad). High-resolution mass spectra (HRMS) were recorded on a quadrupole time-of-flight mass spectrometer (QTOF-MS) using electron spray ionization (ESI) as the ionization method.

Major reagents used include NaCN, toluene, and DMF.

### Example 1: Tafamidis 1

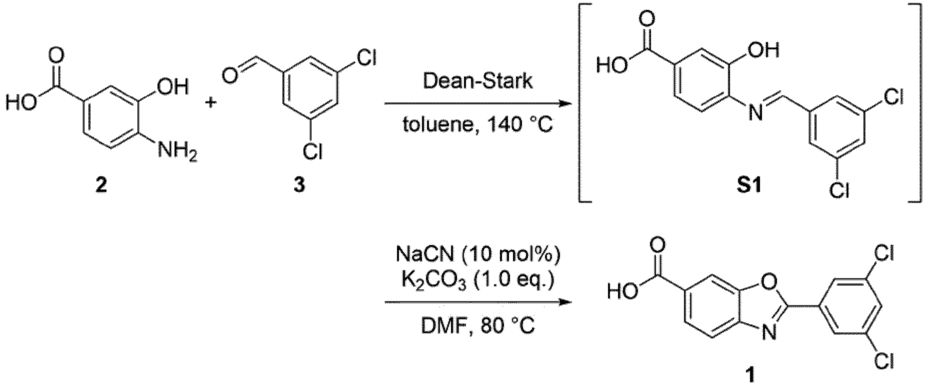

Aniline compound 2 (153 mg, 1.0 mmol) and aldehyde compound 3 (175 mg, 1.0 mmol) were placed in a 2-neck flask equipped with a Dean-Stark apparatus, to which toluene (10 mL) was added. The mixture was stirred at 140°C, and the progress of the reaction was monitored by TLC and ¹H NMR. After complete consumption of compounds 2 and 3, the reaction mixture was concentrated to obtain a mixture of imine compound S1, which was used directly in the next reaction without further separation.

The mixture of imine compound S1 was dissolved in dimethylformamide (2 mL), then potassium carbonate (138 mg, 1.0 mmol) and sodium cyanide (4.9 mg, 0.1 mmol) were added. The mixture was stirred at 80°C, and the progress was monitored by TLC. After complete consumption of compound S1, distilled water (5 mL) was added, and the mixture was stirred for 30 minutes. Then, 1 N HCl solution was added dropwise to the reaction mixture until pH 1 was reached, resulting in the formation of a cream-colored precipitate. The precipitate was filtered, washed with distilled water, and tafamidis 1 (270 mg, 0.87 mmol, 87%) was obtained as a cream-colored solid.

¹H NMR (500 MHz, CD₃ OD) δ 13.29 (br, 1H), 8.29 (d, J = 1.1 Hz, 1H), 8.18 (d, J = 2.0 Hz, 2H), 8.05 (dd, J = 8.4, 1.5 Hz, 1H), 7.98 (t, J = 1.9 Hz, 1H), 7.94 (d, J = 8.4 Hz, 1H)

### Example 2: Tafamidis 1

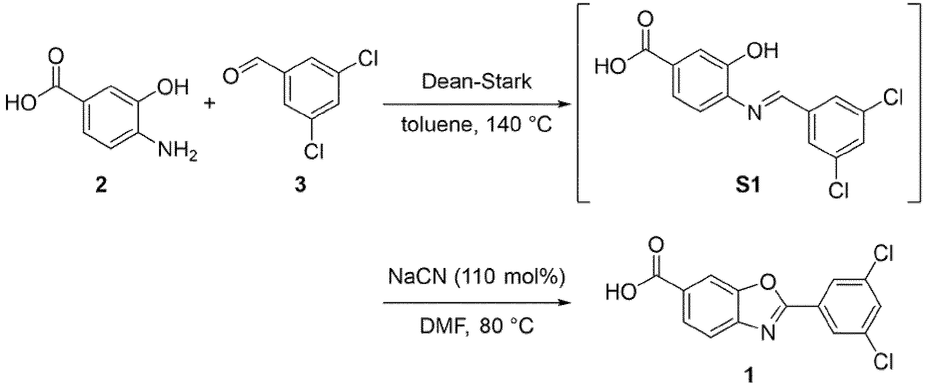

Aniline compound 2 (153 mg, 1.0 mmol) and aldehyde compound 3 (175 mg, 1.0 mmol) were placed in a 2-neck flask equipped with a Dean-Stark apparatus, to which toluene (10 mL) was added. The mixture was stirred at 140°C, and the progress of the reaction was monitored by TLC and ¹H NMR. After complete consumption of compounds 2 and 3, the reaction mixture was concentrated to obtain a mixture of imine compound S1, which was used directly in the next reaction without further separation.

The mixture of imine compound S1 was dissolved in dimethylformamide (2 mL), then sodium cyanide (54 mg, 1.1 mmol) was added. The mixture was stirred at 80°C, and the progress was monitored by TLC. After complete consumption of compound S1, distilled water (5 mL) was added, and the mixture was stirred for 30 minutes. Then, 1 N HCl solution was added dropwise to the reaction mixture until pH 1 was reached, resulting in the formation of a cream-colored precipitate. The precipitate was filtered, washed with distilled water, and tafamidis 1 (270 mg, 0.87 mmol, 87%) was obtained as a cream-colored solid.

¹H NMR (500 MHz, DMSO-d₆) δ 13.29 (br, 1H), 8.29 (d, J = 1.1 Hz, 1H), 8.18 (d, J = 2.0 Hz, 2H), 8.05 (dd, J = 8.4, 1.5 Hz, 1H), 7.98 (t, J = 1.9 Hz, 1H), 7.94 (d, J = 8.4 Hz, 1H)

## Claims

1. A method for manufacturing compound 1 is **characterized by** comprising:
(a) Obtaining compound S1 by reacting compound 2 with compound 3;
(b) Converting compound S1 to compound S1' using 1 equivalent of a base containing M;
(c) Converting compound S1' to compound S2 using M'CN as a catalyst under basic conditions; and
(d) Converting compound S2 to compound 1 by acid treatment; 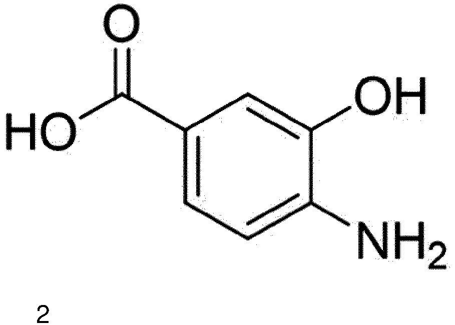 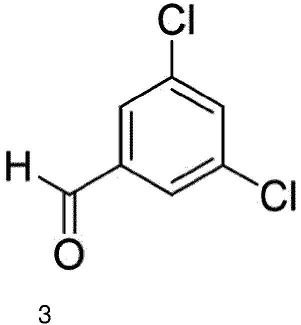 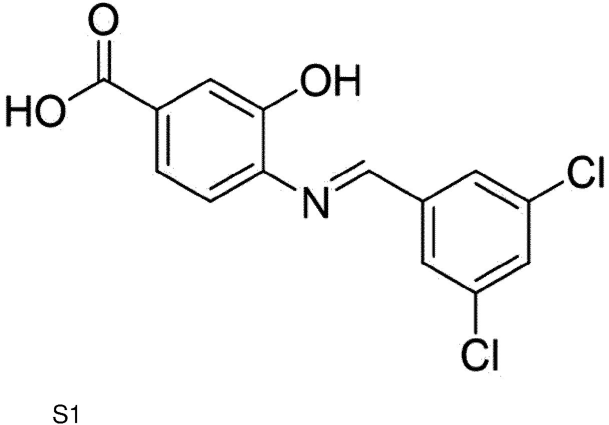 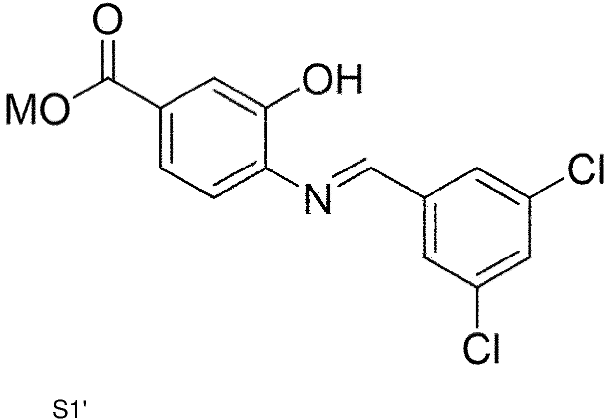 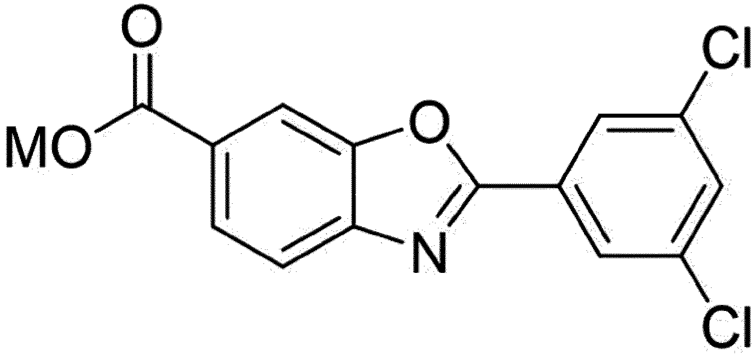 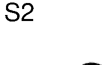 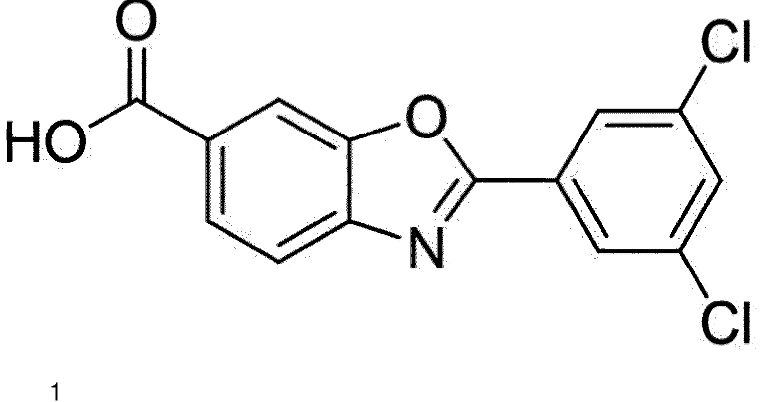 Where M and M' are each independently selected from the group consisting of alkali metals, alkaline earth metals, and ammonium, and the manufacturing method is **characterized by** being performed in air without using metal catalysts and/or additional oxidizing agents.

2. The method for manufacturing of claim 1, wherein step (a) is performed in the presence of a dehydrating agent.

3. The method for manufacturing of claim 2, wherein the dehydrating agent is one or more compounds selected from the group consisting of MgSO₄ and Na₂SO₄, or a molecular sieve.

4. The method for manufacturing of claim 1, wherein step (a) is performed in the presence of TiCl₄ and a base.

5. The method for manufacturing of claim 4, wherein the base is Et3N.

6. The method for manufacturing of claim 1, wherein step (a) is performed using azeotropic distillation.

7. The method for manufacturing of claim 1, wherein step (a) is performed using a Dean-Stark apparatus.

8. The method for manufacturing of claim 1, wherein M is an alkali metal.

9. The method for manufacturing of claim 1, wherein the base containing M is selected from the group consisting of lithium hydroxide, sodium hydroxide, potassium hydroxide, rubidium hydroxide, cesium hydroxide, ammonium hydroxide, lithium carbonate, sodium carbonate, potassium carbonate, rubidium carbonate, cesium carbonate, ammonium carbonate, lithium phosphate, sodium phosphate, potassium phosphate, rubidium phosphate, cesium phosphate, ammonium phosphate, lithium cyanide, sodium cyanide, potassium cyanide, rubidium cyanide, cesium cyanide, ammonium cyanide, lithium acetate, sodium acetate, potassium acetate, rubidium acetate, cesium acetate, and ammonium acetate.

10. The method for manufacturing of claim 9, wherein the base is sodium cyanide.

11. The method for manufacturing of claim 1, wherein M'CN is NaCN or KCN.

12. The method for manufacturing of claim 1, wherein steps (b) and (c) are performed under polar aprotic solvents.

13. The method for manufacturing of claim 12, wherein the polar aprotic solvent is DMF or DMSO.

14. The method for manufacturing of claim 1, wherein step (c) is performed at a temperature ranging from 25°C to 100°C.

15. The method for manufacturing of claim 1, wherein the compound S1 obtained in step (a) is used directly in step (b) without a separate separation process.
